(19) Europäisches Patentamt
European Patent Office
Office européen des brevets

(11) Publication number: 0 115 409
A2

(12) **EUROPEAN PATENT APPLICATION**

(21) Application number: 84300355.9

(22) Date of filing: 20.01.84

(51) Int. Cl.³: C 07 C 103/52
A 61 K 37/02

(30) Priority: 27.01.83 JP 12561/83

(43) Date of publication of application: 08.08.84 Bulletin 84/32

(84) Designated Contracting States: BE CH DE FR GB IT LI NL SE

(71) Applicant: Takeda Chemical Industries, Ltd.
27, Doshomachi 2-chome Higashi-ku
Osaka-shi Osaka, 541(JP)

(72) Inventor: Oka, Yoshikazu
69. Hagiharadaihigashi 1-chome
kawanishi Hyogo 666(JP)

(72) Inventor: Nishikawa, Kohei
5-19, Oharano-kamisatotorimicho
Nishikyo-ku Kyoto 610-11(JP)

(74) Representative: Lewin, John Harvey et al,
Elkington and Fife High Holborn House 52/54 High Holborn
London WC1V 6SH(GB)

(54) **Indane derivatives, their production and use thereof.**

(57) Novel indane derivatives, inclusive of salts thereof, of the formula

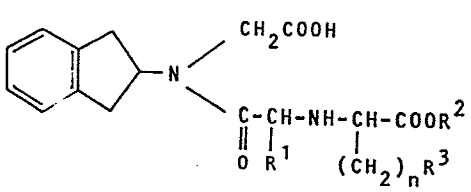

wherein $R^1$ and $R^2$ are independently hydrogen or a lower alkyl group, $R^3$ is a cycloalkyl, bicycloalkyl, tricycloalkyl or benzocycloalkyl group, and n is an integer of 1 to 3, have inhibitory activities of angiotensin converting enzyme, and are useful as antihypertensive agents.

EP 0 115 409 A2

Croydon Printing Company Ltd.

INDANE DERIVATIVES, THEIR PRODUCTION AND USE THEREOF

This invention relates to indane derivatives which are useful as medicines.

More particularly, this invention relates to indane derivatives of the formula;

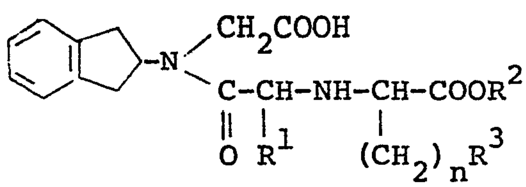

(I)

wherein $R^1$ and $R^2$ are independently hydrogen or a lower alkyl group, $R^3$ is a cycloalkyl, bicycloalkyl, tricycloalkyl or benzocycloalkyl group, and n is an integer of 1 to 3, or salts thereof.

Referring to the above formula (I), as the lower alkyl group representable by $R^1$ or $R^2$, there may be exemplified a $C_{1-4}$ alkyl group such as methyl, ethyl, propyl, isopropyl, butyl, isobutyl or tert-butyl, and, as $R^1$, methyl group is above all, preferable.

As the cycloalkyl group representable by $R^3$, there may be exemplified a $C_{3-8}$ cycloalkyl group such as cyclopropyl, cyclobutyl, cyclopentyl, cyclohexyl, cycloheptyl or cyclooctyl, and, as the bicycloalkyl group, there may be exemplified, for example, norbornyl, bicyclo[2,2,2]-octyl, bicyclo[3,3,1]nonyl or bicyclo[3,3,0]octyl group, and, as the tricycloalkyl group, there may be exemplified adamantyl group, and, as the benzocycloalkyl group, there

may be exemplified 1-indanyl, 2-indanyl, 1,2,3,4-tetrahydro-1-naphthyl, 1,2,3,4-tetrahydro-2-naphthyl or 6,7,8,9-tetrahydro-5H-benzocycloheptyl. As n, 2 is preferable above all.

The salts of compounds (I) include pharmaceutically acceptable salts, for example, inorganic acid salts, such as hydrochloride, hydrobromide, sulfate, nitrate and phosphate; organic acid salts, such as acetate, tartrate, citrate, fumarate, maleate, toluenesulfonate and methanesulfonate; metal salts, such as sodium, potassium, calcium and aluminum salts; and salts with bases, such as ammonium, hydrazine, guanidine, triethylamine, dicyclohexylamine, quinine and cinchonine salts.

The compounds (I) of this invention can be produced, for example, by subjecting a compound of the formula;

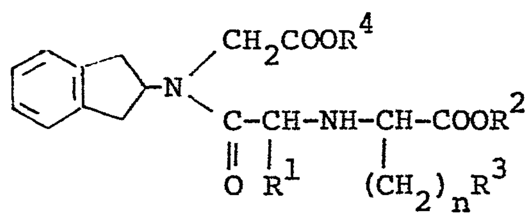

(II)

wherein $R^1$, $R^2$, $R^3$ and n are as defined above, and $R^4$ is a lower alkyl group, to hydrolysis.

The hydrolysis is usually carried out in water, an organic solvent or a mixture thereof, in the presence or absence of an acid such as hydrochloric acid, hydrobromic acid, sulfuric acid, nitric acid, phosphoric acid, acetic acid, trifluoroacetic acid, benzenesulfonic acid, methanesulfonic acid and p-toluenesulfonic acid, or of a base such as ammonia, sodium hydroxide, potassium hydroxide, sodium carbonate, sodium hydrogen carbonate, triethylamine, pyridine and picoline. The reaction temperature is usually in the range of about -20 to +150°C, but, in most cases, the reaction proceeds satisfactorily even at ordinary temperature.

As the lower alkyl group representable by $R^4$, there

may be exemplified those which are similar to those representable by $R^1$ or $R^2$. When, however, $R^2$ is a group other than hydrogen, it is preferable to employ tert-butyl as $R^4$ and to subject the compound to the treatment with an acid for the purpose of removing $R^4$ of (II) selectively.

The compound (II) can be prepared, for example, by subjecting a compound of the formula;

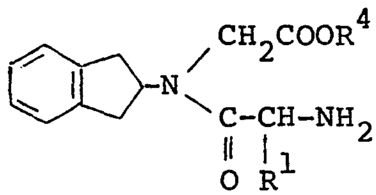

(III)

wherein $R^1$ and $R^4$ are as defined above, and a compound of the formula;

$$R^3(CH_2)_nCOCOOR^2 \qquad (IV)$$

wherein $R^2$, $R^3$ and n are as defined above, to condensation under reductive conditions, or by subjecting a compound of the formula;

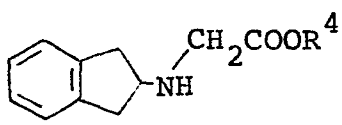

(V)

wherein $R^4$ is as defined above, and a compound of the formula;

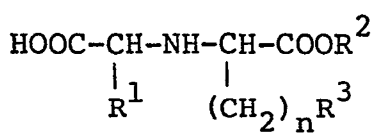

(VI)

wherein $R^1$, $R^2$, $R^3$ and n are as defined above, or a reactive derivative of its carboxyl group, to condensation.

The reductive conditions for the reaction between the compounds (III) and (IV) include reaction conditions used in catalytic reduction using as a catalyst such a metal as platinum, palladium, Raney nickel or rhodium or a mixture thereof with an appropriate carrier; reduction with a metal hydride compound such as lithium aluminum hydride, lithium borohydride, lithium cyanoborohydride, sodium

borohydride or sodium cyanoborohydride; reduction with metallic sodium or metallic magnesium and an alcohol; reduction with such a metal as iron or zinc and such an acid as hydrochloric acid or acetic acid; electrolytic reduction and reduction with a reducing enzyme. The above reaction is usually carried out in the presence of water or an organic solvent (e.g. methanol, ethanol, ethyl ether, dioxane, methylene chloride, chloroform, benzene, toluene, dimethylformamide, dimethylacetamide). The reaction temperature depends on the means of reduction employed, but generally temperatures ranging from -20°C to +100°C are preferred. The reaction can proceed in a satisfactory manner at ordinary pressure, but the reaction may also be carried out under pressure or under reduced pressure according to circumstances.

Referring to the condensation reaction between the compounds (V) and (VI), the reactive derivative of the carboxyl group of the compound (VI) includes acid halide such as acid chloride, acid bromide; acid anhydride which is obtainable by eliminating one molecule of water from 2 molecules of (VI); mixed anhydride obtainable by substituting the hydrogen atom of the carboxyl group with ethoxycarbonyl, isobutyloxycarbonyl, benzyloxycarbonyl, etc. The reaction is generally conducted in a suitable solvent which may be any solvent as far as it does not interfere with the reaction. When (VI) is used as such, i.e. without prior conversing it to a reactive derivative, the reaction is advantageously conducted in the presence of a dehydrating agent such as dicyclohexylcarbodiimide, carbonyldiimidazole, diethyl cyanophosphate, diphenylphosphorylazide, etc. These reactions may also be carried out in the presence of a base such as pyridine, picoline, triethylamine, sodium hydroxide, sodium carbonate, etc. The reaction temperature generally ranges from about -20°C to about +150°C and, in most cases, the reaction proceeds satisfactorily at atmospheric temperature.

Referring to the above formula (I), the compound in which $R^2$ is hydrogen can also be produced by hydrolyzing the compound (I) wherein $R^2$ is a group other than hydrogen. The hydrolysis can be conducted in a similar manner to that of $R^4$ in the above-mentioned (II).

Contrarily, the compound (I) wherein $R^2$ stands for a lower alkyl group can also be produced by subjecting the compound (I) wherein $R^2$ stands for hydrogen to alkylation. The alkylation includes esterification with a corresponding alcohol in a conventional manner; alkylation with, for example, diazomethane, alkyl halide, or alkyl- or aryl-sulfonic acid ester of a corresponding alcohol in a conventional manner; and an addition reaction of an olefin compound such as isobutene.

The starting compound (III) in the above-mentioned production method can be prepared by, for example, a method shown by the following reaction scheme:

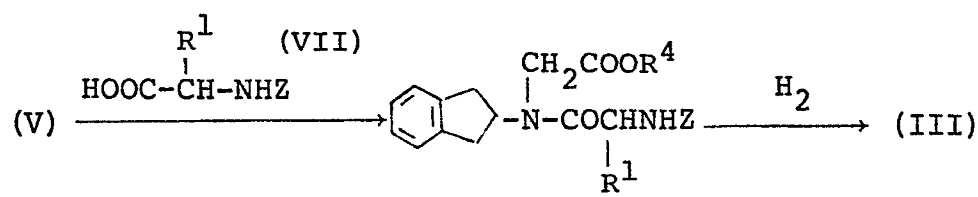

(VII)
(V)
(III)
(VIII)

wherein $R^1$ and $R^4$ are as defined above, and Z is a benzyloxycarbonyl group.

In the above-mentioned reactions, for the reaction between (V) and (VII), similar reaction conditions to those for the reaction between (V) and (VI) are employed, and for the reaction of (VIII) → (III), the reaction conditions for conventional catalytic reduction reaction at ordinary temperature under atmospheric pressure are employed.

The starting compound (VI) can be prepared by, for example, the method shown in the following reaction scheme:

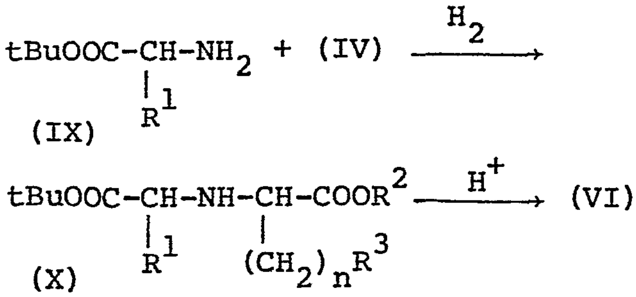


(IX)

(X)

wherein $R^1$, $R^2$, $R^3$ and n are as defined above, and tBu is tert-butyl.

In the above reactions, for the reaction between (IX) and (IV), similar reaction conditions to those for the reaction between (III) and (IV), and for the reaction of (X) to (VI), reaction conditions for conventional acid hydrolysis are employed.

The starting compound (IV) can be prepared by, for example, the method shown in the following reaction scheme:

$$R^3(CH_2)_nCOOC_2H_5 \xrightarrow[2)\ H_3O^+]{1)\ (COOC_2H_5)_2/C_2H_5ONa}$$

$$R^3(CH_2)_nCOCOOH \xrightarrow[H^+]{R^2OH} (IV)$$

wherein $R^2$, $R^3$ and n are as defined above.

The thus obtained objective compound (I) or a salt thereof can be isolated for the reaction mixture by per se conventional separation and purification procedures such as extraction, concentration, neutralization, filtration, recrystallization, column chromatography and thin-layer chromatography.

Since the compound of the formula (I) has an asymmetric carbon atom or atoms in its molecule, plural optical isomers exist. Such isomers, severally or as a mixture, also within the scope of this invention and, if desired, each of the isomers may be produced independently. By conducting the above-mentioned reactions using one of

the isomers of the starting compounds, there can be produced the corresponding optical isomer of (I). When at least one of the starting compounds in racemic, (I) is usually obtained as a mixture of isomers. If desired, the mixture can be separated into the individual isomers by per se conventional separation procedure, for example, through the formation of salts with optically active bases (e.g. cinchonine, cinchonidine, quinine, quinidine, etc.), a variety of chromatographies, fractional recrystallization, etc.

The indane derivatives of the formula (I) and salts thereof show, for example, inhibitory activities to angiotensin converting enzyme and bradykinin decomposing enzyme (Kininase) in animals, especially in mammals (e.g. man, dog, cat, rabbit, guinea pig, rat, etc.), and are useful as diagnostic, prophylactic or therapeutic drugs for hypertension. These compounds are low-toxic, absorbed well on oral administration and stable enough and, therefore, for such clinical applications as mentioned above, can be safely administered orally or parenterally, either as themselves or as formulated with suitable pharmaceutically acceptable carriers, excipients or diluents, in such dosage forms or as such pharmaceutical compositions as powders, granules, tablets, capsules, injections, etc. The dosage depends on the patient's conditions and the route of administration to be used, but, when they are administered to an adult human for the treatment of hypertension, the proper dosage is generally about 0.02 to 20 mg/kg per dose, preferably about 0.2 to 2 mg/kg per dose for oral administration and about 0.002 to 0.2 mg/kg, preferably about 0.02 to 0.2 mg/kg for intravenous injection. About 1 to 3 doses are preferably given daily according to the patient's conditions.

The following reference, working and preparation examples are given to illustrate this invention in further detail but should by no means be construed as limiting the

scope of the invention.

Reference Example 1

Sodium (2.53 g) is weighed in toluene (20 ml). After addition of ethanol (50 ml), the mixture is stirred. After the sodium is completely dissolved, a benzene (100 ml) solution of 3-cyclopentylpropionic acid ethyl ester (17 g) and diethyl oxalate (16 g) is added thereto. The mixture is refluxed for 30 minutes under heating. The reaction mixture is subjected to evaporation on a water bath of 65°C for 30 minutes thereby to remove substances having low boiling point. After cooling, water (300 ml) and petroleum ether (200 ml) are added to the resulting brown syrup and the mixture is shaken well. The aqueous layer is separated, and the petroleum ether layer is extracted three times with 20 ml each of 0.5 N aqueous sodium hydroxide. The aqueous extracts are combined, made acidic with concentrated hydrochloric acid, extracted with ethyl acetate (300 ml). The extract solution is dried over anhydrous magnesium sulfate and subjected to evaporation under reduced pressure to the resultant yellow oily substance are added 10% hydrous dimethylsulfoxide (100 ml) and sodium chloride (8 g). The mixture is stirred at 135-140°C for 1.5 hours. To the cooled reaction mixture is added water (500 ml), and the mixture is extracted with ethyl ether (500 ml). The extract is washed with water (200 ml), dried over anhydrous magnesium sulfate and evaporated under reduced pressure. The resultant brown oily substance is distilled reduced pressure to give ethyl 4-cyclopentyl-2-oxobutylate (9 g, 45%) as a pale yellow oil, b.p. 88-92°C/1.5 mmHg.

Reference Examples 2-4

By employing corresponding 3-substituted propionic acid ethyl ester, similar reaction to that in Reference Example 1 is conducted to give ketoester as shown in Table 1 below.

Table 1

$$R-CH_2CH_2\overset{O}{\overset{\|}{C}}COOC_2H_5$$

| Ref. Ex. No. | R | Yield (%) | b.p.°C/mmHg |
|---|---|---|---|
| 2 | 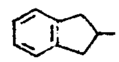 | 56 | 145-155/1 |
| 3 | 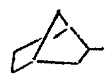 | 21 | 108-111/1 |
| 4 | 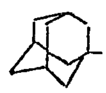 | 45 | 125-130/1 |

Reference Example 5

Raney nickel (wet. 4 g) is washed five times with ethanol (50 ml), to which is added dry ethanol (40 ml). To this mixture is added N-(L-alanyl)-N-(2-indanyl)glycine tert-butyl ester oxalate (4.2 g), and then acetic acid (1.3 g), sodium acetate (1.65 g), molecular sieve 3A (8 g) and ethyl 4-cyclopentyl-2-oxobutylate (2 g) are added in that order. The mixture is subjected to catalytic reduction with stirring vigorously at ordinary temperature and atmospheric pressure. After stoichiometric volume of hydrogen is adsorbed, the catalyst is removed, and ethanol is evaporated under reduced pressure. To the residue are added water (100 ml) and ethyl acetate (300 ml), and the mixture is neutralized with sodium hydrogen carbonate. The ethyl acetate layer is dried over anhydrous magnesium sulfate, followed by evaporation under reduced pressure to obtain an oily substance. This substance is purified by means of a silica-gel column chromatography (hexane: ethyl acetate = 3:1 ∿ 2:1, then hexane: ethyl acetate: acetone = 2:1:1) to give at first N-[N-[(R)-1-ethoxycarbonyl-3-cyclopentylpropyl]-L-alanyl]-N-(2-indanyl)-glycine tert-butyl ester (0.5 g, yield 9.9%) as a yellow oily substance.

IR spectrum (Infrared Spectrum: similar as above):

$\nu_{max}^{neat}$ $cm^{-1}$: 1720(ester), 1650(amido)

Mass spectrum: 500 ($M^+$)

From the subsequent fractions is obtained N-[N-[(S)-1-ethoxycarbonyl-3-cyclopentylpropyl]-L-alanyl]-N-(2-indanyl)glycine tert-butyl ester (2.5 g, 50%) as colorless oily substance.

IR $\nu_{max}^{neat}$ $cm^{-1}$: 1720 (ester), 1640 (amido)

MS (Mass Spectrum: similar as above) m/e: 500 ($M^+$)

## Reference Examples 6-9

By using corresponding ethyl 4-substituted-2-oxobutylate similar reaction to Example 1 is conducted to yield compounds shown in the Table 2 as a colorless oily product.

Table 2

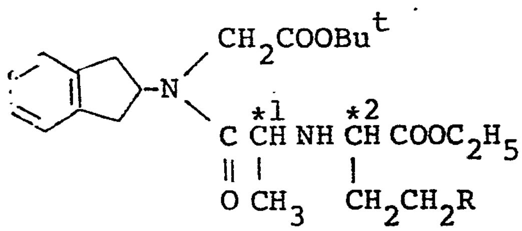

| Ref. Ex. No. | R | Configu-lation *1 | *2 | Yield (%) | IR $\nu_{max}^{neat}$ $cm^{-1}$ Ester | Amido | MS m/e $M^+$ |
|---|---|---|---|---|---|---|---|
| 6 | 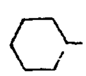 | S | R | 10 | 1740 | 1650 | 514 |
| | | S | S | 50 | 1730 | 1650 | 514 |
| 7 | 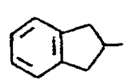 | S | R | 11 | 1740 | 1650 | 548 |
| | | S | S | 63 | 1730 | 1640 | 548 |
| 8 | 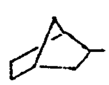 | S | R | 10 | 1740 | 1660 | 526 |
| | | S | S | 45 | 1750 | 1650 | 526 |
| 9 | 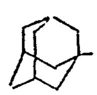 | S | R | 1.8 | 1740 | 1660 | 566 |
| | | S | S | 18 | 1740 | 1650 | 566 |

Example 1

One gram of N-[N-[(S)-1-ethoxycarbonyl-3-cyclopentylpropyl]-L-alanyl]-N-(2-indanyl)glycine tert-butyl ester is dissolved in 12 ml of 3.5 N hydrogen chloride-ethyl acetate solution. The solution is left standing at room temperature for three hours. The reaction solution is concentrated under reduced pressure. To the residue is added 100 ml of ether. The resulting colorless precipitates are collected by filtration to give 0.8 g (83%) of N-[N-(S)-1-ethoxycarbonyl]-3-cyclopentylpropyl]-L-alanyl]-N-(2-indanyl)-glycine hydrochloride, m.p. 159-162°C (decomp.).

IR $\nu_{max}^{nujol}$ $cm^{-1}$: 1750, 1720, 1650 (C=O)

Elemental analysis for $C_{25}H_{36}N_2O_5 \cdot HCl$

Calcd.: C, 62.42; H, 7.75; N, 5.82

Found : C, 62.54; H, 7.77; N, 5.77

$[\alpha]_D^{24.5°}$ + 2.5° (c=1, methanol)

MS m/e: 426 ($M^+-H_2O$)

Example 2

One gram of N-[N-[(S)-1-ethoxycarbonyl-3-cyclohexylpropyl]-L-alanyl]-N-(2-indanyl)glycine tert-butyl ester is dissolved in 12 ml of 3.5 N hydrogen chloride-ethyl acetate solution. The work up of the reaction mixture similarly the case of Example 1 affords 0.7 g of N-[N-[(S)-1-ethoxy carbonyl-3-cyclohexylpropyl]-L-alanyl]-N-(2-indanyl)-glycine hydrochloride.

Melting point: 188-190°C (decomp.)

IR $\nu_{max}^{KBr}$ $cm^{-1}$: 3400(OH), 1740, 1650(amido)

Elemental analysis for $C_{26}H_{38}N_2O_5 \cdot HCl$

Calcd.: C, 63.08; H, 7.94; N, 5.66

Found : C, 62.87; H, 7.94; N, 5.41

MS m/e: 440($M^+-H_2O$), 395, 367, 325, 279

Examples 3-9

Corresponding indanylglycine tert-butyl ester derivatives are treated with hydrogen chloride in a similar manner to that of Example 1 to give respectively. The corresponding compounds, as shown by Table 3 below.

Table 3

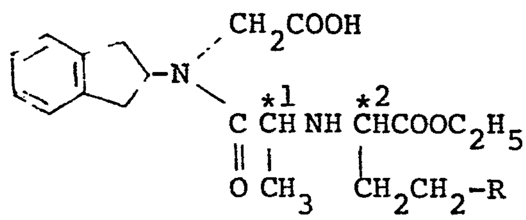

| Ex. No. | R | Configu-ration *1 | *2 | Yield (%) | Melting point (°C, decomp.) | $[\alpha]_D^{24.5°}$ c=1 Methanol | MS m/e ($M^+-H_2O$) |
|---|---|---|---|---|---|---|---|
| 3 | 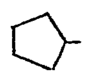 | S | R | 94 | 148-150 | -14.5° | 426 |
| 4 | 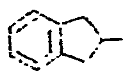 | S | R | 83 | 146-151 | -14.3° | 474 |
| 5 | 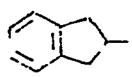 | S | S | 83 | 150-155 | +14.2° | 474 |
| 6 | 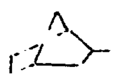 | S | R | 83 | 149-153 | -16.2° | 452 |
| 7 | 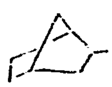 | S | S | 94 | 163-166 | + 5.4° | 452 |
| 8 | 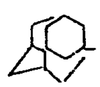 | S | R | 83 | 149-152 | -18.6° | 492 |
| 9 | 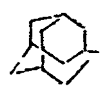 | S | S | 83 | 152-155 | + 6.6° | 492 |

## Preparation Example

The compounds (I) of the present invention are used, for example, for the treatment of hypertension in the following examples of formulation.

1. Tablets

| | | |
|---|---|---|
| (1) | N-[N-[1(S)-ethoxycarbonyl-3-cyclohexylpropyl)-L-alanyl-N-(2-indanyl)glycine hydrochloride | 10 g |
| (2) | Lactose | 90 g |
| (3) | Corn Starch | 29 g |

| | | |
|---|---|---|
| (4) | Magnesium Stearate | 1 g |
| | | 130 g |

for 1000 tablets

The above ingredients (1), (2) and 17 g of corn starch are blended, and granulated using a paste prepared from 7 g of corn starch. Five grams of corn starch and the ingredient (4) are added to the resulting granules and the mixture is compressed by a tabletting machine to prepare 1000 tablets having a diameter 7 mm each containing 10 mg of the active ingredient (1).

2. Capsules

| | | |
|---|---|---|
| (1) | N-[N-[1(S)-ethoxycarbonyl-3-(2-indanyl)propyl]-L-alanyl]-N-(2-indanyl)glycine hydrochloride | 10 g |
| (2) | Lactose | 135 g |
| (3) | Cellulose Fine Powder | 70 g |
| (4) | Magnesium Stearate | 5 g |
| | | 220 g |

for 1000 capsules

All of the above components are blended and encapsulated into Gelatin Capsule No. 3 (X Japanese Pharmacopoiea) to prepare 1000 capsules each containing 10 mg of the active component (1).

3. Injectable Solution

| | | |
|---|---|---|
| (1) | N-[N-[1(S)-ethoxycarbonyl-3-cyclopentylpropyl]-L-alanyl]-N-(2-indanyl)glycine hydrochloride | 10 g |
| (2) | Sodium Chloride | 9 g |
| (3) | Chlorobutanol | 5 g |

All of the above ingredients are dissolved in 1000 ml of distilled water and charged into 1000 brown ampules each containing 1 ml of the solution. The ampules are replaced with nitrogen gas and sealed. The entire preparation steps are conducted under sterile conditions.

What we claim is:

1. A compound of the formula

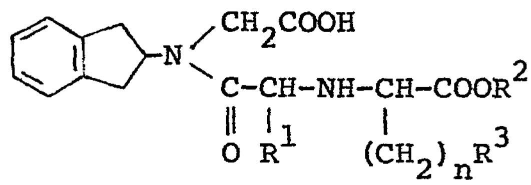

wherein $R^1$ and $R^2$ are independently hydrogen or $C_{1-4}$ alkyl, $R^3$ is cycloalkyl, bicycloalkyl, tricycloalkyl or benzocycloalkyl and n is an integer of 1 to 3, or a pharmaceutically acceptable salt thereof.

2. A compound according to Claim 1, wherein $R^1$ is $C_{1-4}$ alkyl.

3. A compound according to Claim 1, wherein $R^1$ is methyl.

4. A compound according to Claim 1, wherein $R^2$ is $C_{1-4}$ alkyl.

5. A compound according to Claim 1, wherein $R^2$ is ethyl.

6. A compound according to Claim 1, wherein $R^3$ is $C_{3-8}$ cycloalkyl.

7. A compound according to Claim 1, wherein $R^3$ is cyclopentyl or cyclohexyl.

8. A compound according to Claim 1, wherein $R^3$ is cyclohexyl.

9. A compound according to Claim 1, wherein n is 2.

10. The compound according to Claim 1, which is N-[N-1-ethoxycarbonyl-3-cyclopentylpropyl]-alanyl]-N-(2-

indanyl)glycine.

11. The compound according to Claim 1, which is N-[N-1-ethoxycarbonyl-3-cyclohexylpropyl]-alanyl]-N-(2-indanyl)-glycine.

12. The compound according to Claim 1, which is N-[N-(S)-1-ethoxycarbonyl-3-cyclohexylpropyl]-L-alanyl]-N-(2-indanyl)-glycine.

13. The compound according to Claim 1, which is N-[N-(S)-1-ethoxycarbonyl-3-cyclohexylpropyl]-L-alanyl]-N-(2-indanyl)glycine hydrochloride.

14. A pharmaceutical composition suitable for prevention or treatment of hypertension which comprises, as an active ingredient, an effective antihypertensive amount of a compound as claimed in any one of Claims 1 to 13 in association with a pharmaceutically acceptable carrier, excipient or diluent therefor.

15. A compound as claimed in any one of Claims 1 to 13 or a pharmaceutical composition as claimed in Claim 14 for use in therapeutical treatment of a warm-blooded animal.

16. A process for producing a compound of the formula (I)

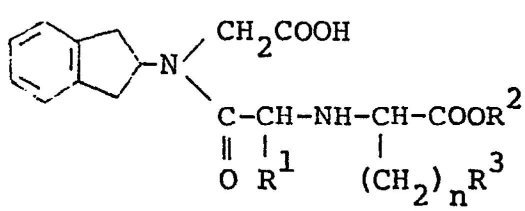

wherein $R^1$ and $R^2$ are independently hydrogen or $C_{1-4}$ alkyl, $R^3$ is cycloalkyl, bicycloalkyl, tricycloalkyl or benzocycloalkyl and n is an integer of 1 to 3,

or a pharmaceutically acceptable salt thereof, which comprises

(a) hydrolyzing a compound of the formula

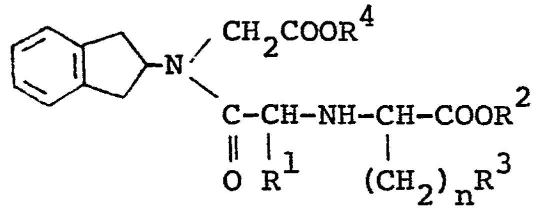

wherein $R^1$, $R^2$, $R^3$ and n are as defined above, and $R^4$ is $C_{1-4}$ alkyl, and

(b) if desired, converting the thus obtained compound of the formula (I) into a pharmaceutically acceptable salt thereof.

17. A process according to Claim 16, wherein $R^4$ is tert-butyl.